# EUROPÄISCHE PATENTSCHRIFT

(11) **EP 0 545 198 B1**
(45) Veröffentlichungstag und Bekanntmachung des Hinweises auf die Patenterteilung: **15.09.1999**
(21) Anmeldenummer: 92119913.9
(22) Anmeldetag: 23.11.1992
(51) Int. Cl.: C07K 7/06, C07K 7/08, C07K 9/00, C07K 5/08, A61K 38/00

(54) **Peptide mit insulinartiger Wirkung**
Peptides with insulin like activity
Peptides ayant une activité comme insuline

(30) Priorität: 29.11.1991 DE 4139376
(43) Veröffentlichungstag der Anmeldung: 09.06.1993
(73) Patentinhaber: HOECHST AKTIENGESELLSCHAFT, 65926 Frankfurt am Main (DE)
(72) Erfinder: Frick, Wendelin, Dr., W-6274 Hünstetten (DE); Müller, Günter, Dr., W-6231 Sulzbach/Ts. (DE); Müllner, Stefan, Dr., W-6203 Hochheim am Main (DE); Breipohl, Gerhard, Dr., W-6000 Frankfurt am Main 71 (DE)

(56) Entgegenhaltungen:
- EP-A- 0 455 833
- EP-A- 0 491 361
- EP-A- 0 532 915

## Beschreibung

Die Erfindung betrifft Peptide mit insulinartiger Wirkung, die sich zur Behandlung des Diabetes mellitus eignen.

Insuline bestehen aus zwei Polypeptidketten, der A-Kette, die 21 Aminosäurereste enthält, und der B-Kette mit 30 Aminosäureresten. A- und B-Kette sind über zwei Disulfidbrücken miteinander verbunden, wobei die Cysteinreste in Position A7 und B7 sowie A20 und B19 miteinander verknüpft sind. Eine dritte Disulfidbrücke besteht zwischen A6 und A11. Tierische und menschliche Insuline werden in den Pankreata in Form von Präproinsulinen gebildet. Menschliches Präproinsulin besteht aus einem Präpeptid mit 24 Aminosäureresten, daran anschließend ein Proinsulin mit 86 Aminosäureresten mit der folgenden Konfiguration: Präpeptid-B-Arg-Arg-C-Lys-Arg-A, wobei C für eine Aminosäurekette mit 31 Resten steht. Während der Exkretion aus den Langerhansschen Inseln wird das Präpeptid abgespalten, und es entsteht Proinsulin. Abschließend wird die C-Kette proteolytisch gespalten, und es entsteht das wirksame menschliche Insulin.

Insulin übt eine Vielzahl von Wirkungen auf insulinsensitives Gewebe aus. Ein auffälliger Effekt ist die schnelle Reduktion des Glucosespiegels im Blut von Säugetieren, wenn Insulin angewendet wird. Dies wird durch eine schnelle Aufnahme der Glucose aus dem Blut durch Muskel- und Fettzellen bewirkt. Insulin aktiviert ferner die Glycogen-Synthetase und inhibiert die Lipolyse. Insulin fördert die Proteinsynthese aus Aminosäuren und verstärkt die Induktion von Glycokinase und Phosphofruktokinase und inhibiert die Bildung von bestimmten Enzymen der Glyconeogenese wie Pyruvatcarboxylase und Fruktosediphosphatase.

Der Diabetes Typ II, nicht insulinabhängiger Diabetes, geht einher mit einer Insulinresistenz der peripheren Gewebe, wie Muskel- oder Fettgewebe. Die dadurch reduzierte Glucoseverwertung wird verursacht durch fehlende Insulinstimulation des Glucosetransports und nachfolgender metabolischer Prozesse. Diese multiple Resistenz läßt auf einen Defekt auf Rezeptor- oder Post-Rezeptor-Ebene, d.h. vor der Erzeugung der "second messenger", schließen (Garvey, Diabetes/Metabolism Reviews, 5, (1989), 727-742).

Peptide mit insulinartiger Wirkung sind bereits in EP-A-491361 und DE-A-5 920 783 vorgeschlagen worden.

In dem Bestreben weitere wirksame Peptide mit insulinartiger Wirkung zu finden, wurde nun gefunden, daß die erfindungsgemäßen Peptide in vitro insulinartige Wirkung zeigen, eine gute Serumstabilität aufweisen und auch an insulinresistenten Geweben insulinartige Wirkung zeigen und sich so zur Behandlung von Diabetes mellitus eignen.

Die Erfindung betrifft daher Peptide mit insulinartiger Wirkung der Formel I 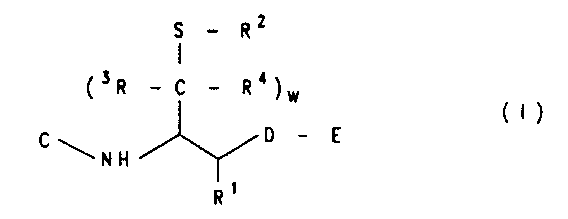 wobei
- C ist:: a) Wasserstoffatom,
b) ein Rest aus der Gruppe: Aminosäure substituiert durch Acetyl, Penicillinamin, Thiophenaminosäure, Tyr, Asn, Phe, Trp, Nal, D-Tyr, D-Asn oder Gluconsäure,
- D ist:: ein Rest aus der Gruppe:
Asp, Asn, D-Asp, D-Asn, Tyr, D-Tyr, Glucosamin oder kovalente Bindung,
- E ist:: a) -NH-(CH₂)ₙ-NR⁵₂,
   worin R⁵ gleich oder verschieden ist und ein Rest aus der Gruppe ist:
   1) Wasserstoffatom,
   2) 1 bis 6 Zuckerreste oder
   3) 1 bis 6 Zuckerreste, ein- oder mehrfach unabhängig voneinander substituiert durch:
      3.1 Methyl,
      3.2 Zuckerrest,
      3.3 Dizuckerrest,
      3.4 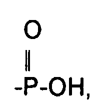
      3.5 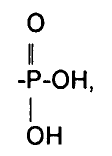
      3.6 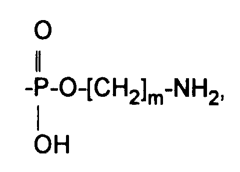
      3.7 Inositol,
      3.8 Inositolphosphat oder
      3.9 Phosphatrest mit Phosphatschutzgruppe,
      n ist eine ganze Zahl von 0 bis 6,
      m ist eine ganze Zahl von 0 bis 4,
b) Glycerinrest,
c) -NH-(CH₂)ₘ-R⁶-R⁷,
worin R⁶ ist:
   1) -O-,
   2) -CO-O-,
   3) 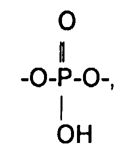
   4) 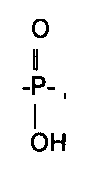
   5) -NH-CO-O-,
   6) -S-,
   7) -SO,
   8) -SO₂,
   9) -SO₃ oder
   10) -O-CO-O-,
worin R⁷ ist:
   1) Wasserstoffatom,
   2) 1 bis 6 Zuckerreste oder
   3) 1 bis 6 Zuckerreste, ein- oder mehrfach unabhängig voneinander substituiert durch
      3.1 Methyl,
      3.2 Zuckerrest,
      3.3 Dizuckerrest,
      3.4 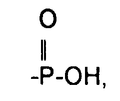
      3.5 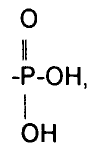
      3.6 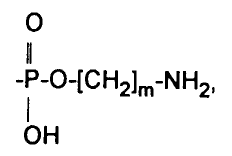
      3.7 Inositol,
      3.8 Inositolphosphat,
      3.9 Phosphatrest mit Phosphatschutzgruppe oder
      3.10 -[CH₂]ₘ-CN,
      m ist eine ganze Zahl von 0 bis 4,
- R¹ ist:: a) -(C₁-C₄)-Alkyl,
b) =O oder
c) -O-(C₁-C₄)-Alkyl,
- R² ist:: a) eine Schwefelschutzgruppe,
b) -SO₂,
c) -SO₃,
d) -(C₁-C₃)-Alkyl,
e) ein O₂-Rest,
f) ein O₃-Rest,
g) 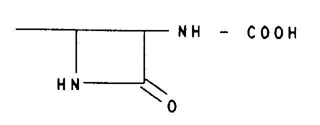 oder
h) Wasserstoffatom,
- R³ oder R⁴: sind unabhängig voneinander
a) Wasserstoffatom oder
b) Methyl,
- W: ist eine ganze Zahl 1 oder 2,
stereoisomere Formen, Dimere der Peptide der Formel I mit Cystin als Dimerisierungskomponente oder physiologisch verträgliche Salze des Peptids der Formel I,
mit Ausnahme von Peptiden der Formel I
worin bedeuten
- C: ein Wasserstoffatom,
- R¹: = O,
- R²: eine Schwefelschutzgruppe oder ein Wasserstoffatom,
- R³ und R⁴: ein Wasserstoffatom,
- E: -NH-(CH₂)ₘ-R⁶-R⁷,
- R⁶: -CO-O-
- R⁷: ein Wasserstoffatom und
- m und w: 1.

Bevorzugt sind Peptide der Formel I, wobei
C ist ein Rest aus der Gruppe:
   Aminosäure substituiert durch Acetyl, Penicillamin, Thiophenaminosäure, Tyr, Asn, Phe, Trp, Nal, D-Tyr, D-Asn oder Gluconsäure,
D ist ein Rest aus der Gruppe:
   Asp, Asn, D-Asp, D-Asn, Tyr, D-Tyr, Glucosamin oder kovalente Bindung,
   - E ist:: a) -NH-(CH₂)ₙ-NR⁵₂,
      worin R⁵ gleich oder verschieden ist und ein Rest aus der Gruppe:
      1) Wasserstoffatom,
      2) Glucose,
      3) Gluconsäure,
      4) Galaktonsäure,
      5) Mannonsäure,
      6) Glucosamin,
      7) Mannose,
      8) Fructose,
      9) Galaktose,
      10) Dimannose oder
      11) Trimannose,
      n ist eine ganze Zahl von 0 bis 4,
   b) -NH-(CH₂)ₘ-R⁶-R⁷,
      worin R⁶ ist
      1) -O-,
      2) -O-CO-O-,
      3) 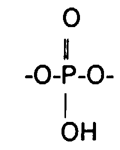 oder
      4) -NH-CO-O-,
      worin R⁷ ist:
      1) Wasserstoffatom oder
      2) definiert wie unter a)1) bis d)11),
      m ist eine ganze Zahl von 1 bis 3,
   - R¹ ist:: a) =O oder
   b) Methyl,
   - R² ist:: a) Wasserstoffatom,
   b) Methyl oder
   c) -SO₃,
W ist eine ganze Zahl 1 oder 2,
stereoisomere Formen oder physiologisch verträgliche Salze des Peptids der Formel I.

Besonders bevorzugt sind Peptide der Formel I, wobei
C ist ein Rest aus der Gruppe Tyr, Asn, D-Tyr, D-Asn, 2-(2-Thienyl)-glycin, 3-(2-Thienyl)-alanin oder Phe,
D ist ein Rest aus der Gruppe Asn, Tyr, D-Asn oder D-Tyr,
E ist ein Rest aus der Gruppe Ethyldiamin oder Ethanolamin,
W ist 1 oder 2,
R¹ ist =O,
R² ist ein Wasserstoffatom oder Methyl,
stereoisomere Formen oder physiologisch verträgliche Salze des Peptids der Formel I.

Unter dem Begriff Alkyl werden geradkettige oder verzweigte Kohlenwasserstoffketten verstanden.

Mit dem Begriff Aminosäuren bzw. Aminosäurereste sind z.B. die stereoisomeren Formen, d.h. D- oder L-Formen, folgender Verbindungen gemeint:

| | | |
|---|---|---|
| Alanin | Glycin | Prolin |
| Cystein | Histidin | Glutamin |
| Asparaginsäure | Isoleucin | Arginin |
| Glutaminsäure | Lysin | Serin |
| Phenylalanin | Leucin | Threonin |
| Tryptophan | Methionin | Valin |
| Tyrosin | Asparagin | |

| | |
|---|---|
| 2-Aminoadipinsäure | 2-Aminoisobuttersäure |
| 3-Aminoadipinsäure | 3-Aminoisobuttersäure |
| beta-Alanin | 2-Aminopimelinsäure |
| 2-Aminobuttersäure | 2,4-Diaminobuttersäure |
| 4-Aminobuttersäure | Desmosin |
| Piperidinsäure | 2,2-Diaminopimelinsäure |
| 6-Aminocapronsäure | 2,3-Diaminopropionsäure |
| 2-Aminoheptansäure | N-Ethylglycin |
| 2-(2-Thienyl)-glycin | 3-(2-Thienyl)-alanin |
| Penicillamin | Sarkosin |
| N-Ethylasparagin | N-Methylisoleucin |
| Hydroxylysin | 6-N-Methyllysin |
| allo-Hydroxylysin | N-Methylvalin |
| 3-Hydroxyprolin | Norvalin |
| 4-Hydroxyprolin | Norleucin |
| Isodesmosin | Ornithin |
| allo-lsoleucin | |
| N-Methylglycin | |

Die Kurzschreibweise der Aminosäuren erfolgte nach der allgemein üblichen Schreibweise (vgl. Schröder, Lübke, The Peptides, Band I, New York 1965, Seiten XXII-XXIII; Houben-Weyl, Methoden der Organischen Chemie, Band XV/1 und 2, Stuttgart 1974). Die Aminosäure pGlu steht für Pyroglutamyl, Nal für 3-(2-Naphthyl)-alanin, Azagly-NH₂ für eine Verbindung der Formel NH₂-NH-CONH₂ und D-Asp für die D-Form von Asparaginsäure. Unter dem Begriff Phosphoaminosäuren werden Aminosäuren verstanden, deren Carboxylgruppe durch eine Phosphatgruppe ausgetauscht wurde. Peptide sind ihrer chemischen Natur nach Säureamide und zerfallen bei der Hydrolyse in Aminosäuren.

Die Bindung zwischen C - für den Fall, daß es sich um eine Aminosäure handelt - und der Verbindung der Formel II 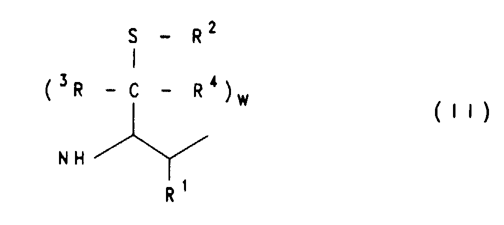 wobei R¹, R², R³, R⁴ und W die Bedeutung wie in der Formel I haben ist eine Peptidbindung. Die Bindungen zwischen
a) D und E,
b) D und der Verbindung der Formel II oder
c) E und der Verbindung der Formel II
sind - falls es sich bei D um eine Aminosäure, bzw. Aminosäurerest, handelt - jeweils Peptidbindungen.

Unter Zucker werden Aldosen und Ketosen mit 3 bis 7 Kohlenstoffatomen verstanden, die der D- oder L-Reihe angehören können; dazu gehören auch Aminozucker oder Uronsäuren. Beispielhaft seien genannt Glucose, Mannose, Fructose, Galaktose, Ribose, Erythrose, Glycerinaldehyd, Sedoheptulose, Glucosamin, Galaktosamin, Glucuronsäure, Galakturonsäure, Gluconsäure, Galaktonsäure oder Mannonsäure.

Mit Dizucker sind Saccharide gemeint, die aus zwei Zuckereinheiten bestehen.

Di-, Tri- oder Oligosaccharide entstehen durch acetalartige Bindung von 2 oder mehreren Zuckern. Die Bindungen können dabei in der α- oder β-Form auftreten. Die Bindungen zwischen den Zuckern erfolgen bevorzugt über C-Atom 1 und C-Atom 6, C-Atom 1 und C-Atom 2 sowie C-Atom 1 und C-Atom 4 der jeweiligen Zucker. Die α-Form der Bindung zwischen den Zuckern ist bevorzugt.

Wenn der Zucker substituiert ist, so erfolgt die Substitution bevorzugt am Wasserstoffatom einer OH-Gruppe des Zuckers.

Die erfindungsgemäßen Verbindungen können eine oder mehrere Phosphatgruppen enthalten, die auch noch mit einer Phosphatschutzgruppe derivatisiert sein können. Phosphatschutzgruppen sind beispielsweise Phenyl, Benzyl oder Hydroxypropylnitril (Houben Weyl, Methoden der Organischen Chemie, Band 12/1 oder Band 12/2; Teiheimer, Synthetic Methods of Organic Chemistry, Vol 45).

Unter dem Begriff insulinresistentes Gewebe werden beispielsweise Rattenfettzellen verstanden, die keinen Insulinrezeptor mehr besitzen.

Unter physiologisch verträglichen Salzen der Verbindung der Formel I sind insbesondere pharmazeutisch verwendbare oder nicht-toxische Salze zu verstehen. Solche Salze werden z.B. von Verbindungen der Formel I, welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z.B. Triethylamin und Tris-(2-hydroxy-ethyl)-amin. Verbindungen der Formel I, welche basische Gruppen, z.B. eine Aminogruppe oder eine Guanidinogruppe enthalten, bilden mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure Salze. Verbindungen, in denen basische und saure Gruppen in gleicher Zahl vorliegen, bilden innere Salze und sind auf eine dritte Salzkomponente nicht angewiesen.

Die erfindungsgemäßen Peptide werden nach den allgemeinen Methoden der Peptidchemie stufenweise vom C-terminalen Ende her oder durch Kupplung von Segmenten hergestellt (Houben-Weyl, Methoden der Organischen Chemie, Band 15/1,2). Die Peptidkupplungen können z.B. nach der Methode der gemischten Anhydride, über Aktivester, Azide oder nach der Carbodiimid-Methode, insbesondere unter Zusatz reaktionsbeschleunigender und racemisierungsverhindernder Substanzen, wie z.B. 1-Hydroxybenzotriazol, N-Hydroxysuccinimid, 3-Hydroxy-4-oxo-3.4-dihydro-1.2.3-benzotriazin, N-Hydroxy-5-norbornen-2.3-dicarboximid, ferner unter Verwendung aktiver Derivate des 1-Hydroxybenzotriazols oder Anhydriden von Phosphor-, Phosphon- und Phosphinsäuren bei einer Reaktionstemperatur zwischen -10°C und dem Siedepunkt des Lösungsmittels, vorzugsweise zwischen -5°C und 40° C durchgeführt werden.

Geeignete Lösungsmittel dafür sind Dimethylformamid, Dimethylacetamid, N-Methylpyrrolidon oder Dimethylsulfoxid. Sofern die Löslichkeit der Komponenten es erlaubt, können auch Lösungsmittel wie Methylenchlorid, Chloroform oder Tetrahydrofuran eingesetzt werden. Die genannten Methoden sind z.B. in Meinhofer-Gross: "The Peptides" Academic Press, Vol. I, (1979) beschrieben.

Falls zur Verhinderung von Nebenreaktionen oder für die Synthese spezieller Peptide erforderlich, sind die funktionellen Gruppen in der Seitenkette von Aminosäuren durch geeignete Schutzgruppen (siehe z.B. T.W. Greene, "Protective Groups in Organic Synthesis") zusätzlich geschützt, wobei in erster Linie Arg(Tos), Arg(Mts), Arg(Mtr), Arg(PMV), Asp(OBzl), Asp(OBut), Cys(4-MeBzl), Cys(Acm), Cys(SBut), Glu(Obzl), Glu(Obut), His(Tos), His(Fmoc), His(Dnp), His(Trt), Lys(CI-Z), Lys(Boc), Met(O), Ser(Bzl), Ser(But), Thr(Bzl), Thr(But), Trp(Mts), Trp(CHO), Tyr(Br-Z), Tyr(Bzl) oder Tyr(But) eingesetzt werden.

Als Aminoschutzgruppen werden bevorzugt der durch katalytische Hydrierung abspaltbare Benzyloxycarbonyl-(Z-)Rest, der durch schwache Säuren abspaltbare 2-(3,5-Dimethyloxyphenyl)propyl(2)oxycarbonyl (Ddz-) oder Trityl- (Trt)-Rest und der durch sekundäre Amine abspaltbare 9-Fluorenylmethyloxycarbonyl- (Fmoc)-Rest herangezogen. Die SH-Gruppe des Cysteins kann durch eine Reihe von Schutzgruppen blockiert sein. Bevorzugt wird hier der Trityl-(Trt)-Rest und der S-tert.-Butyl(StBu-)Rest. Der Tritylrest kann durch Jod-Oxydation unter Bildung der Cystin-Verbindungen oder durch reduzierende saure Spaltung zu den Cystein-Verbindungen abgespalten werden (Liebigs Ann. Chem. 1979, 227-247).

Der S-tert.-Butyl-Rest wird dagegen am besten mit Tributylphosphin reduktiv gespalten (Aust. J. Chem. 19 (1966) 2355-2360). OH- und COOH-Funktionen in den Seitenketten werden am besten durch den sauer abspaltbaren tert.-Butyl-(tBu-)Rest geschützt (siehe auch: Meienhofer-Gross: "The Peptides", Vol. 3).

Die Oligosaccharide werden nach bekannten Verfahren hergestellt (H. Paulsen, Angew. Chem. Int. Ed. 21 (1982) S. 155). Bevorzugt wird die Trichloracetimidat Methode zur Synthese von Oligosacchariden angewendet (R. R. Schmidt, Angew. Chem. Int. Ed. 25 (1986) 212 - 235; T. Ogawa, Tetrahedron Lett. 31 (1990) 2439 - 2442).

Die Synthese der Phosphate erfolgt mit Hilfe der Phosphittriester-Methode (W. Bannwarth, Helv. Chim. Acta 70 (1987), 175 - 186), bevorzugt wird der erste Reaktionsschritt mit dem Peptidanteil des Peptides der Formel I durchgeführt, der zweite Reaktionsschritt mit dem Oligosaccharidanteil.

Für die Hydroxygruppen der Zucker kommen im wesentlichen folgende Schutzgruppen in Frage: Benzyl-, Acetyl-, Benzoyl-, Pivaloyl-, Trityl-, tert.-Butyldimethylsilyl-, Benzyliden- oder Isopropylidenschutzgruppen.

Die Verbindungen der Formel I und deren physiologisch verträgliche Salze dienen in erster Linie als Wirkstoffe für pharmazeutische Zubereitung zur Behandlung des Diabetes mellitus oder nicht insulinabhängiger Diabetes, wobei die pharmazeutische Zubereitung sich durch einen Gehalt an mindestens einer Verbindung der Formel I und/oder mindestens einem von dessen physiologisch verträglichen Salzen in gelöster, amorpher und/oder kristalliner - vorzugsweise in amorpher und/oder kristalliner Form auszeichnet.

Die pharmazeutische Zubereitung ist vorzugsweise eine Lösung oder Suspension zu Injektionszwecken mit einem pH-Wert von etwa 3,0 bis 9,0, vorzugsweise von etwa 5,0 bis 8,5, welche ein geeignetes Isotoniemittel, ein geeignetes Konservierungsmittel und gegebenenfalls einen geeigneten Puffer, sowie gegebenenfalls auch ein Depotprinzip, alles in steriler wäßriger Lösung oder Suspension, enthält. Die Gesamtheit der Zubereitungsbestandteile außer dem Wirkstoff bildet den Zubereitungsträger.

Geeignete Isotoniemittel sind z.B. Glycerin, Glucose, Mannit, NaCl, Calcium- oder Magnesium-Verbindungen wie z.B. CaCl₂ oder MgCl₂.

Geeignete Konservierungsmittel sind z.B. Phenol, m-Cresol, Benzylalkohol und/oder p-Hydroxybenzoesäureester.

Als Puffersubstanzen, insbesondere zur Einstellung eines pH-Wertes von etwa 5,0 bis 8,5 können z.B. Natriumacetat, Natriumcitrat oder Natriumphosphat verwendet werden. Ansonsten sind zur Einstellung des pH-Wertes auch physiologisch unbedenkliche verdünnte Säuren (typischerweise HCI) bzw. Laugen (typischerweise NaOH) geeignet.

Zwecks Variation des Wirkungsprofils der erfindungsgemäßen Zubereitung können auch modifizierte (vgl. EP-B 132 769 und EP-B 132 770) und/oder unmodifizierte Insuline, vorzugsweise Rinder-, Schweine- oder Humaninsulin, insbesondere Humaninsulin, zugemischt werden.

Die pharmazeutische Zubereitung wird dadurch hergestellt, daß man mindestens eine Verbindung der Formel I und/oder mindestens eines von dessen physiologisch verträglichen Salzen, gegebenenfalls zusammen mit modifizierten und/oder unmodifizierten Insulin(derivat)en, mit einem physiologisch unbedenklichen Träger sowie gegebenenfalls mit geeigneten Zusatz- und Hilfsstoffen in eine geeignete Darreichungsform bringt.

Die Erfindung wird nun durch die folgenden Beispiele näher erläutert.

### Beispiel 1 H-L-Tyr-L-Cys-L-Asn-NH-CH₂-CH₂OH

1 a) Z-Asn-NH-CH₂-CH₂-OAc (2):
   8,0 g (20.6 mmol) Z-AsnONp 1 werden in 200 ml Pyridin gelöst und mit 5 g (81.8 mmol) Ethanolamin und 5 ml N-Ethylmorpholin versetzt. Nach 16 Stunden Stehen bei Raumtemperatur wird unter Rühren bei 5°C 50 ml Essigsäureanhydrid zugetropft. Die Reaktionsmischung wird noch 2 Stunden bei Raumtemperatur gerührt und dann am Hochvakuum eingeengt. Der Rückstand wird heiß in 150 ml Methanol gelöst und eingeengt. Das Produkt kristallisiert nach Zugabe von 100 ml Methylenchlorid/Methanol (15:1) und 200 ml n-Heptan/Ethylacetat (2:1). Die Ausbeute beträgt 6.1 g (84 %) weiße Kristalle vom Schmp. 175°C. DC [Methylenchlorid/Methanol (9:1)] R_{f} = 0.7. C₁₆H₂₁N₃O₆ (351,36).
1b) H-Asn-NH-CH₂-CH₂-OAC (3):
   Zu einer Lösung von 12,0 g (34.0 mmol) 2 in 200 ml Methanol/Essigsäure (1:1) gibt man 2,0 g Palladium/Kohle (10 % Pd) und hydriert 2 Stunden bei Raumtemperatur. Die Lösung wird über Kieselgel filtriert, eingeengt und der Rückstand mit Flash-Chromatographie [Methylenchlorid/Methanol/konz. Ammoniak (30/5/1)] gereinigt. Ausbeute 7.3 g (98 %) gelbliches Öl. DC [Methylenchlorid/Methanol/konz. Ammoniak (3015/1)] R_{f} = 0.5. C₈H₁₅N₃O₄ (217.23).
1c) Trt-Cys-(Trt)-Asn-NH-CH₂-CH₂-OAC (4):
   Zu einer Lösung von 0,8 g (3.7 mmol) 3 und 2,8 g (4.5 mmol) Trt-Cys-(Trt)-OH in DMF gibt man bei 0°C unter Rühren 1,5 g (4.5 mmol) TOTU1 [o-((Cyano-(ethoxycarbonyl)methyliden)amino-1,1,3,3-tetramethyluronium-tetrafluoroborat], 0,64 g (4.5 mmol) Oxim [Ethyl-(hydroxyimino)cyanoacetat] und 1,7 ml (13.5 mmol) N-Ethylmorpholin und läßt 2 Stunden bei 0°C rühren. Nach Zugabe von 200 ml Ethylacetat wird 3mal mit gesättigter NaHCO₃-Lösung gewaschen, über MgSO₄ getrocknet und eingeengt. Der Rückstand wird mit n-Heptan/Ethylacetat (6:1) verrieben. Ausbeute 2,2 g (74 %) weiße Kristalle. Schmp. 185°C. DC [Methylenchlorid/Methanol (15:1)] R_{f} = 0.4. C₄₉H₄₈N₄O₅S (805.0).
1d) H-Cys-(Trt)-Asn-NH-CH₂-CH₂OH (5):
   4.0 g (5.0 mmol) 4 werden in 200 ml CH₂Cl₂ gelöst. Dazu gibt man 4 ml Wasser und 3 ml Trifluoressigsäure. Nach 15 Minuten wird 3mal mit gesättigter NaHCO₃-Lösung gewaschen, über MgSO₄ getrocknet und eingeengt. Der Rückstand wird in 50 ml absolutem Methanol gelöst und 0,5 ml 1 M Natriummethanolat-Lösung zugetropft. Nach 15 Minuten wird 50 ml Methylenchlorid zugegeben und über Kieselgel filtriert. Nach Einengen des Lösungsmittels wird der Rückstand mit Flash-Chromatographie [Methylenchlorid/Methanol (9:1)] gereinigt. Ausbeute 2,2 g (85 %) weißer amorpher Feststoff. DC [Methylenchlorid/Methanol (5:1)] R_{f} = 0.7. C₂₈H₃₂N₄O₄S (520.6).
1e) Boc-Tyr-(Boc)-Cys-(Trt)-Asn-NH-CH₂-CH₂OH (6):
   2,2 g (4.2 mmol) 5, 2,1 g (5.5 mmol) Boc Tyr (Boc)OH, 1,8 g (5.5 mmol) TOTU, 0,8 g (5.5 mmol) Oxim und 1,4 ml (11 mmol) N-Ethylmorpholin werden analog der Darstellung von 4 umgesetzt. Ausbeute 2,6 g (70 %) weiße Kristalle. Schmp. 169 - 170°C. DC [Methylenchlorid/Methanol (15:1)] R_{f} = 0.3. C₄₇H₅₇N₅O₁₀S (884.07).
1f) H-Tyr-Cys-Asn-NH-CH₂-CH₂OH (7):
   2,0 g (2.3 mmol) 6 werden in einer Mischung aus 10 ml Trifluoressigsäure und 10 ml Ethylmercaptan gelöst. Nach 4 Stunden schüttet man den Ansatz in 100 ml Wasser. Die wäßrige Phase wird 3mal mit Ether extrahiert und eingeengt. Der Rückstand wird mit Flash-Chromatographie [Methylenchlorid/Methanol/konz. Ammoniak (30/15/5)] gereinigt. Ausbeute 0,85 g (85 %). DC [Methylenchlorid/Methanol/konz. Ammoniak (30/15/5)]. R_{f} = 0.3 C₁₈H₂₇N₅O₆S (441,51). MS (M+H⁺) = 442,5.

### Beispiel 14 H-Tyr-Cys-AsnNH-CH₂-CH₂-O-PO(OH)-O-6Manα1-OMe

### 14 a) Trt-Cys-(Trt)-AsnNH-CH₂-CH₂-O-PO(O-CH₂-CH₂-CN)-O-6Man(2,3,4-tribenzyl)α1-OMe (8) (Me steht für Methylrest, Man steht für Mannoserest)

1,1 g (2,37 mmol) Methyl-2,3,4-tri-O-benzyl-α-D-mannopyranosid (A. Vasella, Helv. Chim. Acta 62 (1979), 2400 - 2410) wird in 15 ml trockenem Acetonitril gelöst. Dazu gibt man 1,36 g (4,50 mmol) Bis-(diisopropylamino)-(2-cyanethoxy)-phosphin (Aldrich) und 170 mg (2,40 mmol) Tetrazol (2mal aus Ethylacetat umkristallisiert). Nach 20 Minuten wird über wenig Kieselgel mit n-Heptan/Ethylacetat (2 : 1) filtriert und das Filtrat eingeengt.

Der ölige Rückstand (2,0 g) wird in 10 ml trockenem Acetonitril und 8 ml trockenem Methylenchlorid gelöst und eine Lösung von 1,5 g (1,97 mmol) Trt-Cys-(Trt)-AsnNH-CH₂-CH₂-OH in 5 ml trockenem Tetrahydrofuran zugegeben. Nach Zugabe von 175 mg (2,5 mmol) Tetrazol läßt man die Lösung 2 Std. bei Raumtemperatur stehen. Dann wird über wenig Kieselgel mit Methylenchlorid/Methanol (15 : 1) filtriert und das Filtrat eingeengt (3 g Rohprodukt).

Das Rohprodukt wird in 50 ml Methylenchlorid gelöst und auf 0 °C gekühlt. Nach Zugabe von 500 mg meta-Chlor-perbenzoesäure rührt man 20 Minuten bei 20 °C, verdünnt mit 200 ml Methylenchlorid und wäscht 3mal mit gesättigter NaHCO₃-Lösung. Die organische Phase wird über MgSO₄ getrocknet, filtriert, eingeengt und der Rückstand mit Flash-Chromatographie [Methylenchlorid/Methanol (15 : 1)] gereinigt.
Ausbeute 1,2 g (45 % über 3 Stufen).
Dünnschichtchromatographie (DC) [Methylenchlorid/Methanol (15 : 1)]; R_{f} = 0,3. C₇₈H₈₀N₅O₁₂PS (1342,61).
MS (M+Li⁺) = 1348,4.

### 14 b) H-Cys-(Trt)-Asn-NH-CH₂-CH₂-O-PO(O-CH₂-CH₂-CN)-O-6Man(2,3,4-tribenzyl)α1-OMe (9)

1,2 g (0,89 mmol) 8 werden in 70 ml Methylenchlorid gelöst. Dazu gibt man 1 ml Wasser und 1 ml Trifluoressigsäure. Nach 15 Min. wird 3 mal mit gesättigter NaHCO₃-Lösung gewaschen, über MgSO₄ getrocknet und eingeengt. Der Rückstand wird mit Flash-Chromatographie [Methylenchlorid/Methanol (15 : 1)]; gereinigt. Ausbeute 0,79 g (80 %); DC [Methylenchlorid/Methanol (9 : 1)]; R_{f} = 0,60. C₅₉H₆₆N₅O₁₂PS (1100,28). MS (M+Li⁺) = 1106,4.

### 14 c) Z-Tyr-(Bn)-Cys-(Trt)-AsnNH-CH₂-CH₂-O-PO(O-CH₂-CH₂-CN)-O-6Man(2,3,4-tribenzyl)-α1-OMe (10)

300 mg (0,27 mmol) 9, 325 mg (0,80 mmol) Z-Tyr-(Bn)-OH (Bachem), 260 mg (0,80 mmol) TOTU, 115 mg (0,80 mmol) Oxim und 0,3 ml (2,5 mmol) N-Ethylmorpholin werden analog der Darstellung von 4 (Beispiel 1 c) umgesetzt. Ausbeute 320 mg (79 %) weißer Feststoff. DC [MethylenchloridlMethanol (9 : 1)]; R_{f} = 0,65.

### 14 d) H-Tyr-Cys-AsnNH-CH₂-CH₂-O-PO(OH)-O-6Manα1-OMe (11)

Bei - 78 °C werden 40 ml Ammoniak verflüssigt und mit 100 mg Natrium versetzt. Nach 15 Minuten Rühren werden 20 ml trockenes Tetrahydrofuran (THF) zugegeben. Dann wird eine Lösung von 120 mg (0,080 mmol) 10 in 10 ml trockenem THF zugetropft. Die Lösung wird bei - 78 °C 30 Minuten gerührt. Zur Aufarbeitung wird mit Ammoniumchlorid versetzt bis die blaue Farbe verschwindet. Dann werden 20 ml Methanol und 10 ml Wasser vorsichtig zugegeben. Die Lösung wird eingeengt und der Rückstand mit Flash-Chromatographie [Methylenchlorid/Methanol/konz. Ammoniak (3 : 3 : 1)] gereinigt. Ausbeute 40 mg (71 %) weißer Feststoff.

DC [Methylenchlorid/Methanol/konz. Ammoniak (3 : 3 : 1)]; R_{f} = 0,2 - 0,3. C₂₅H₄₀N₅O₁₄PS (697,68). MS (M+H⁺) = 698,3.

Entsprechend den Reaktionsschritten von Beispiel 1 oder 14 werden die nachfolgenden Verbindungen hergestellt:

**Tabelle 1**

| Beispiel Nr. | Verbindung | Summenformel Mol-Gewicht | Massenspektrum Molpeak (M+H⁺) |
|---|---|---|---|
| 1 | H-L-Tyr-L-Cys-L-Asn-NH-CH₂-CH₂-OH | C₁₈H₂₇N₅O₆S 441,5 | 442,5 |
| 2 | H-L-Tyr-L-Cys-NH-CH₂-CH₂-NH₂ | C₁₄H₂₂N₄O₃S 326,4 | 327,4 |
| 3 | H-L-Asn-L-Cys-L-Tyr-NH-CH₂-CH₂OH | C₁₈H₂₇N₅O₆S 441,5 | 442,5 |
| 4 | H-D-Tyr-L-Cys-L-Asn-NH-CH₂-CH₂OH | C₁₈H₂₇N₅O₆S 441,5 | 442,5 |
| 5 | H-L-Cys-L-Asn-NH-CH₂-CH₂OH | C₉H₁₈N₄O₄S 278,3 | 279,3 |
| 6 | H-L-Cys-L-Tyr-NH-CH₂-CH₂OH | C₁₄H₂₁N₃O₄S 327,4 | 328,4 |
| 7 | H-L-Tyr-L-Cys-NH-(CH₂)₄-NH₂ | C₁₆H₂₆N₄O₃S 354,5 | 355,5 |
| 8 | H-L-Tyr-L-Cys-L-Asn-NH-CH₂-CH₂-NH-Gluconsäure | C₂₄H₃₈N₆O₁₁S 618,7 | 619,7 |
| 9 | H-L-Tyr-L-Cys-L-Asn-NH-CH₂-CH₂-NH₂ | C₁₈H₂₈N₆O₅S 440,5 | 441,5 |
| 10 | Acetyl-L-Tyr-L-Cys-L-Asn-NH-CH₂-CH₂OH | C₂₀H₂₉N₅O₇S 483,6 | 484,6 |
| 11 | H-L-Tyr-L-Cys-L-Asn-NH-(CH₂)₂-O-PO(OH)-O(CH₂)₂-CN | C₂₁H₃₁N₆O₉PS 574,2 | 575,2 |
| 12 | H-L-Tyr-L-Cys-L-Asn-NH-(CH₂)₂-O-PO(OH)-O-6Manα1-O-CH₃ | C₂₅H₄₀N₅O₁₄PS 697,6 | 698,3 |
| 13 | H-L-Tyr-L-Cys-L-Asn-NH-(CH₂)₂-O-PO(OH)-O-6Manα1-2Manα1-O-CH₃ | C₃₁H₅₀N₅O₁₉PS 859,3 | 860,3 |
| 14 | H-L-Tyr-L-Cys-L-Asn-NH-(CH₂)₂-O-PO(OH)-O-6Manα1-2-Manα1-6Manα1-O-CH₃ | C₃₇H₆₀N₅O₂₄PS 1021,7 | 1022,7 |

In Tabelle 1 steht Man für den Zucker Mannose

Die biologische Aktivität der erfindungsgemäßen Peptide der Formel I wird anhand von präparativ isolierten Fettzellen und Diaphragma-Stücken aus der Ratte bestimmt.

Die Präparation von Fettzellen aus der Ratte erfolgte wie folgt:
Fettgewebe des Nebenhodens (Wistar-Ratte, 160 - 180 g, keine Futterbeschränkung) wird mit Kollagenase verdaut und die entstandenen vereinzelten Fettzellen werden durch Flotation mehrmals gewaschen.

Präparation von Diaphragma-Stücken aus der Ratte:
Aus mehrmals gewaschenen Hemi-Diaphragmen (Wistar-Ratte, 60 - 70 g, keine Futterbeschränkung) werden kleine Gewebsstücke (5 mm Durchmesser) gestanzt.

### A) Glycogenese

Dieser Test bestimmt die insulinstimulierbare Glycogensynthese in Muskelzellen, welche den Glucosetransport über die Plasmamembran und die Umwandlung der Glucose in Glycogen einschließlich der funktionellen Insulin-Signalübertragungskaskade umfaßt.

Diaphragmastücke werden in Krebs-Ringer-Henselight-Puffer (KRH-Puffer) mit 50 µM D-[U-¹⁴C]Glucose in Gegenwart oder Abwesenheit von Insulin oder den erfindungsgemäßen Peptiden für 15 min bei 37°C inkubiert. Nach Absaugen des Mediums werden die Gewebestücke intensiv gewaschen, bei -70°C eingefroren und nachfolgend bei 2°C in einem Polytron-Zerkleinerer homogenisiert. Das Homogenat wird zentrifugiert (2000 g) und der Überstand auf Filterpapier pipettiert. Zur Bestimmung des gebildeten Glycogens werden die Filter in Trichloressigsäure (TCA) (5 %) überführt, mit Ethanol und Aceton gewaschen, getrocknet und ihre Radioaktivität über Szintillationsmessung ([¹⁴C]Glycogen[dpm*10⁻³] bestimmt.

### B) Lipogenese

Dieser Test bestimmt die insulinstimulierbare Umwandlung von Glucose in Toluollösliche Produkte (Triglyzeride, Phospholipide, Fettsäuren), welche den GlucoseTransport und die Triglyzerid (Glycerin-3-P-Synthese, Veresterung)-/Phospholipid-/Fettsäure-Synthese einschließlich der Insulin-Signalübertragungskaskade erfordert.

Ratten-Fettzellen in KRH-Puffer werden mit D-[3-³H]Glucose (0,2 mM oder 1 mM Endkonz.) in Gegenwart oder Abwesenheit von Insulin oder erfindungsgemäßer Peptide für 90 min bei 37°C inkubiert. Durch Zugabe eines Toluol-löslichen Szintillationscocktails werden die Zellen aufgeschlossen und die Lipide von wasserlöslichen Produkten und dem Inkubationsmedium abgetrennt. Nach Phasentrennung wird die in Lipide inkorporierte Radioaktivität durch Szintillationsmessung direkt ohne Entfernung der wäßrigen Phase bestimmt ([³H]Lipid [dpm*10⁻³]).

### C) Glucosetransportaktivität

Isolierte Plasmamembranvesikel werden aus Rattenfettzellen gewonnen, in dem die Fettzellen zweimal in Homogenisierungspuffer (20 mM Trihydroxyaminomethan (Tris)/HCI, pH 7,4, 1 mM, 4°C, EDTA, 0,25 M Saccharose) gewaschen und dann in 20 ml des gleichen Puffers homogenisiert werden (Glas-Homogenisator mit Teflon-Pistill).

Das Homogenisat wird zentrifugiert (16000 x g, 15 min), das Sediment wird im gleichen Puffer suspendiert und erneut zentrifugiert. Das Sediment wird in 5 ml Homogenisierungspuffer suspendiert und auf ein Saccharose-Kissen (1,12 M Saccharose, 20 Mm Tris/HCI, pH 7,4, 1 mM EDTA) geschichtet, nach Zentrifugation (100000 x g, 70 min) wird die Interphase mit den Plasmamembranvesikeln mit einer Spritze entnommen, mit 45 ml Puffer verdünnt und erneut zentrifugiert (48000 x g, 45 min). Das Sediment wird in 10 ml Puffer suspendiert, erneut zentrifugiert und in 3 ml Puffer erneut suspendiert.

Isolierte Plasmamembranvesikel werden in Gegenwart oder Abwesenheit von Insulin oder den erfindungsgemäßen Peptiden für 30 min bei 25°C inkubiert. Nachfolgend werden die Vesikel mit 50 µM D-[3-³H]-Glucose und L-(1-¹⁴C]Glucose der gleichen spezifischen Radioaktivität für 10 sec bei 25°C inkubiert. Die Mischungen werden rasch über Nitrozellulose-Filter abgesaugt. Die Filter werden ausgiebig gewaschen und getrocknet. Ihre Radioaktivität wird durch Flüssigszintillationsmessung bestimmt. Der spezifische Transport (D-[³H]Glucose/L-[¹⁴C]Glucose [dpm*10⁻³]) wird als Differenz zwischen der [³H]- und [¹⁴C]-Radioaktivität errechnet.

Die Tabelle 2 zeigt die Ergebnisse in Prozent der maximalen Insulinwirkung. Die Wirkung von Humaninsulin (HI) wird mit 100 % angegeben. Die Konzentrationsangaben der eingesetzten erfindungsgemäßen Peptide beziehen sich auf die Konzentration bei der 25 % bzw. 20 % der maximalen Hl-Wirkung erreicht wird.

**Tabelle 2**

| Verbindung Beispiel Nr. | Lipogenese % der max. HI-Wirkung | Konzentration [µM] bei der 25 % der max. HI-Wirkung erreicht wird | Glucosetransport % der max. HI-Wirkung | Konzentration [µM] bei der 20 % der max. HI-Wirkung erreicht wird | Glycogenese % der max. HI-Wirkung | Konzentration [µM] bei der 25 % der max. HI-Wirkung erreicht wird |
|---|---|---|---|---|---|---|
| 1 | 35 | 375 | 42 | 240 | 26 | 550 |
| 2 | 32 | 500 | 40 | 350 | 28 | 650 |
| 3 | 31 | 375 | 38 | 225 | 42 | 285 |
| 4 | 31 | 410 | 38 | 260 | 23 | - |
| 5 | 21 | - | 25 | 600 | 16 | - |
| 6 | 17 | - | 24 | 750 | 32 | 480 |
| 7 | 18 | - | 10 | - | 2 | - |
| 8 | 12 | - | 15 | - | 11 | - |
| 9 | 30 | 330 | 42 | 300 | 25 | 450 |
| 10 | 36 | 200 | 40 | 260 | 45 | 240 |
| 11 | 33 | 120 | 36 | 140 | 31 | 100 |
| 12 | 37 | 95 | 40 | 105 | 32 | 90 |
| 13 | 35 | 142 | 32 | 122 | 29 | 139 |
| 14 | 24 | 125 | 29 | 152 | 25 | 135 |

## Patentansprüche

1. Peptid mit insulinartiger Wirkung der Formel I, 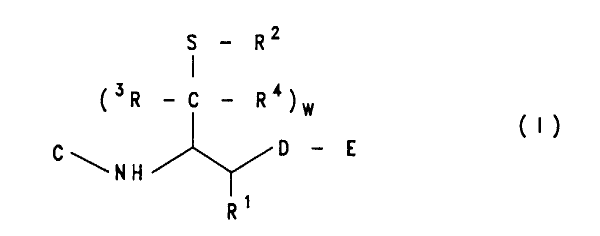 wobei
C ist:
a) Wasserstoffatom,
b) ein Rest aus der Gruppe: Aminosäurerest substituiert durch Acetyl, Penicillinamin, Thiophenaminosäure, Tyr, Asn, Phe, Trp, Nal, D-Tyr, D-Asn oder Gluconsäure,
D ist: ein Rest aus der Gruppe: Asp, Asn, D-Asp, D-Asn, Tyr, D-Tyr, Glucosamin oder kovalente Bindung,
E ist:
a) -NH-(CH₂)ₙ-NR⁵₂,
worin R⁵ gleich oder verschieden ist und ein Rest aus der Gruppe ist:
1) Wasserstoffatom,
2) 1 bis 6 Zuckerreste oder
3) 1 bis 6 Zuckerreste, ein- oder mehrfach unabhängig voneinander substituiert durch:
3.1 Methyl,
3.2 Zuckerrest,
3.3 Dizuckerrest,
3.4 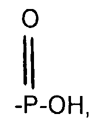
3.5 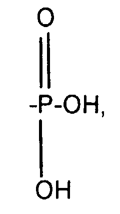
3.6 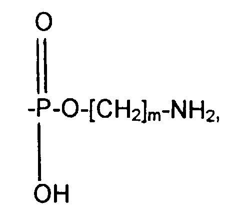
3.7 Inositol,
3.8 Inositolphosphat oder
3.9 Phosphatrest mit Phosphatschutzgruppe,
n ist eine ganze Zahl von 0 bis 6,
m ist eine ganze Zahl von 0 bis 4,
b) Glycerinrest,
c) -NH-(CH₂)ₘ-R⁶-R⁷,
worin R⁶ ist:
1) -O-,
2) -CO-O-,
3) 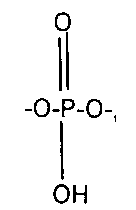
4) 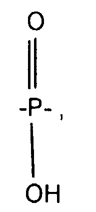
5) -NH-CO-O-,
6) -S-,
7) -SO,
8) -SO₂,
9) -SO₃ oder
10) -O-CO-O-,
worin R⁷ ist:
1) Wasserstoffatom,
2) 1 bis 6 Zuckerreste oder
3) 1 bis 6 Zuckerreste, ein- oder mehrfach unabhängig voneinander substituiert durch
3.1 Methyl,
3.2 Zuckerrest,
3.3 Dizuckerrest,
3.4 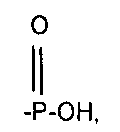
3.5 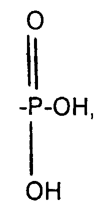
3.6 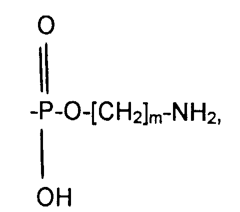
3.7 Inositol,
3.8 Inositolphosphat,
3.9 Phosphatrest mit Phosphatschutzgruppe oder
3.10 -[CH₂]ₘ-CN,
m ist eine ganze Zahl von 0 bis 4,
R¹ ist:
a) -(C₁-C₄)-Alkyl,
b) =O oder
c) -O-(C₁-C₄)-Alkyl,
R² ist: a) eine Schwefelschutzgruppe,
b) -SO₂,
c) -SO₃,
d) -(C₁-C₃)-Alkyl,
e) ein O₂-Rest,
f) eine O₃-Rest,
g) 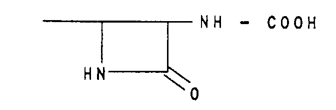 oder
h) Wasserstoffatom,
R³ oder R⁴ sind unabhängig voneinander
a) Wasserstoffatom oder
b) Methyl,
W ist eine ganze Zahl 1 oder 2,
stereoisomere Formen, Dimere der Peptide der Formel I mit Cystin als Dimerisierungskomponente oder physiologisch verträgliche Salze des Peptids der Formel I,
mit Ausnahme eines Peptids der Formel I,
worin bedeuten
C ein Wasserstoffatom,
R¹ = O,
R² eine Schwefelschutzgruppe oder ein Wasserstoffatom,
R³ und R⁴ ein Wasserstoffatom,
E -NH-(CH₂)ₘ-R⁶-R⁷,
R⁶ -CO-O-,
R⁷ ein Wasserstoffatom und
m und w 1.

2. Peptid nach Anspruch 1, dadurch gekennzeichnet, daß
C ist ein Rest aus der Gruppe:
Aminosäure substituiert durch Acetyl, Penicillamin, Thiophenaminosäure, Tyr, Asn, Phe, Trp, Nal, D-Tyr, D-Asn oder Gluconsäure,
D ist ein Rest aus der Gruppe:
Asp, Asn, D-Asp, D-Asn, Tyr, D-Tyr, Glucosamin oder kovalente Bindung,
E ist:
a) -NH-(CH₂)ₙ-NR⁵₂,
worin R⁵ gleich oder verschieden ist und ein Rest aus der Gruppe:
1) Wasserstoffatom,
2) Glucose,
3) Gluconsäure,
4) Galaktonsäure,
5) Mannonsäure,
6) Glucosamin,
7) Mannose,
8) Fructose,
9) Galaktose,
10) Dimannose oder
11) Trimannose,
n ist eine ganze Zahl von 0 bis 4, oder
b) -NH-(CH₂)ₘ-R⁶-R⁷,
worin R⁶ ist
1) -O-,
2) -O-CO-O-,
3) 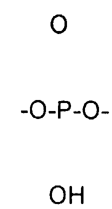 oder
4) -NH-CO-O-,
worin R⁷ ist:
1) Wasserstoffatom oder
2) definiert wie unter a)1) bis d)11),
m ist eine ganze Zahl von 1 bis 3,
R¹ ist:
a) =O oder
b) Methyl,
R² ist:
a) Wasserstoffatom,
b) Methyl oder
c) -SO₃,
W ist eine ganze Zahl 1 oder 2,
stereoisomere Formen oder physiologisch verträgliche Salze des Peptids der Formel I.

3. Peptid nach Anspruch 1 oder 2, dadurch gekennzeichnet, daß
C ist ein Rest aus der Gruppe Tyr, Asn, D-Tyr, D-Asn, 2-(2-Thienyl)-glycin, 3-(2-Thienyl)-alanin oder Phe,
D ist ein Rest aus der Gruppe Asn, Tyr, D-Asn oder D-Tyr,
E ist ein Rest aus der Gruppe Ethyldiamin oder Ethanolamin,
W ist 1 oder 2,
R¹ ist =O,
R² ist ein Wasserstoffatom oder Methyl,
stereoisomere Formen oder physiologisch verträgliche Salze des Peptids der Formel I.

## Claims

1. A peptide with an insulin-like action, of formula I, 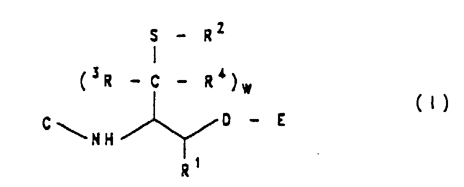 in which
C is:
a) a hydrogen atom,
b) a residue selected from the group consisting of:
an amino acid residue substituted by acetyl, penicillamine, thiopheneamino acid, Tyr, Asn, Phe, Trp, Nal, D-Tyr, D-Asn or gluconic acid,
D is: a residue selected from the group consisting of:
Asp, Asn, D-Asp, D-Asn, Tyr, D-Tyr, glucosamine or a covalent bond,
E is:
a) -NH-(CH₂)ₙ-NR⁵₂,
in which R⁵, which is identical or different,
is a radical selected from the group consisting of:
1) a hydrogen atom,
2) 1 to 6 sugar residues, and
3) 1 to 6 sugar residues monosubstituted or polysubstituted independently of one another by:
3.1 methyl,
3.2 a sugar residue,
3.3 a disugar residue,
3.4 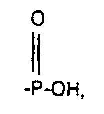
3.5 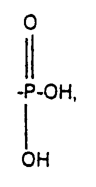
3.6 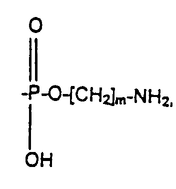
3.7 inositol,
3.8 inositol phosphate, or
3.9 a phosphate residue with a phosphate protecting group,
n is an integer from 0 to 6, and
m is an integer from 0 to 4,
b) a glycerol residue, or
c) -NH-(CH₂)ₘ-R⁶-R⁷,
in which R⁶ is:
1) -O-,
2) -CO-O-,
3 ) 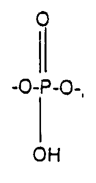
4) 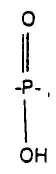
5) -NH-CO-O-,
6) -S-,
7) -SO,
8) -SO₂,
9) -SO₃, or
10) -O-CO-O-,
in which R⁷ is:
1) a hydrogen atom,
2) 1 to 6 sugar residues, or
3) 1 to 6 sugar residues monosubstituted or polysubstituted independently of one another by
3.1 methyl,
3.2 a sugar residue,
3.3 a disugar residue,
3.4 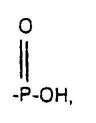
3.5 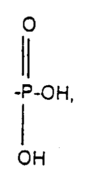
3.6 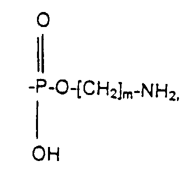
3.7 inositol,
3.8 inositol phosphate,
3.9 a phosphate residue with a phosphate protecting group, or
3.10 -[CH₂]ₘ-CN,
m is an integer from 0 to 4,
R¹ is:
a) -(C₁-C₄)-alkyl,
b) =O, or
c) -O- (C₁-C₄) -alkyl,
R² is:
a) a sulfur protecting group,
b) -SO₂,
c) -SO₃,
d) -(C₁-C₃) -alkyl,
e) an O₂ radical,
f) an O₃ radical,
g) 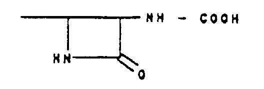 or
h) a hydrogen atom,
R³ or R⁴ independently of one another are
a) a hydrogen atom, or
b) methyl, and
W is an integer 1 or 2,
stereoisomeric forms, dimers of the peptides of formula I with cystine as the dimerization component, or physiologically acceptable salts of the peptide of formula I,
with the exception of a peptide of formula I, in which
C is a hydrogen atom,
R¹ is =O,
R² is a sulfur protecting group or a hydrogen atom,
R³ and R⁴ are a hydrogen atom,
E is -NH-(CH₂)ₘ-R⁶-R⁷,
R⁶ is -CO-O-,
R⁷ is a hydrogen atom, and
m and w are 1.

2. A peptide as claimed in claim 1, wherein
C is a residue selected from the group consisting of:
an amino acid substituted by acetyl, penicillamine, thiopheneamino acid, Tyr, Asn, Phe, Trp, Nal, D-Tyr, D-Asn and gluconic acid,
D is a radical selected from the group consisting of:
Asp, Asn, D-Asp, D-Asn, Tyr, D-Tyr, glucosamine and a covalent bond,
E is:
a) -NH-(CH₂)ₙ-NR⁵₂,
in which R⁵, which is identical or different,
is a radical selected from the group consisting of:
1) a hydrogen atom,
2) glucose,
3) gluconic acid,
4) galactonic acid,
5) mannonic acid,
6) glucosamine,
7) mannose,
8) fructose,
9) galactose,
10) dimannose, and
11) trimannose,
n is an integer from 0 to 4, or
b) -NH-(CH₂)ₘ-R⁶-R⁷,
in which R⁶ is
1) -O-,
2) -O-CO-O-,
3) 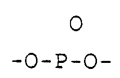 or
4) -NH-CO-O-,
in which R⁷ is:
1) a hydrogen atom, or
2) defined as under a)1) to a)11), and
m is an integer from 1 to 3,
R¹ is:
a) =O, or
b) methyl,
R² is:
a) a hydrogen atom,
b) methyl, or
c) -SO₃, and
W is an integer 1 or 2,
stereoisomeric forms or physiologically tolerable salts of the peptide of formula I.

3. A peptide as claimed in claim 1 or 2, wherein
C is a residue selected from the group consisting of Tyr, Asn, D-Tyr, D-Asn, 2-(thien-2-yl)glycine, 3-(thien-2-yl)alanine and Phe,
D is a residue selected from the group consisting of Asn, Tyr, D-Asn and D-Tyr,
E is a radical selected from the group consisting of ethylenediamine and ethanolamine,
W is 1 or 2,
R¹ is =O, and
R² is a hydrogen atom or methyl,
stereoisomeric forms or physiologically tolerable salts of the peptide of formula I.

## Revendications

1. Peptide à activité de type insuline, de formule I, 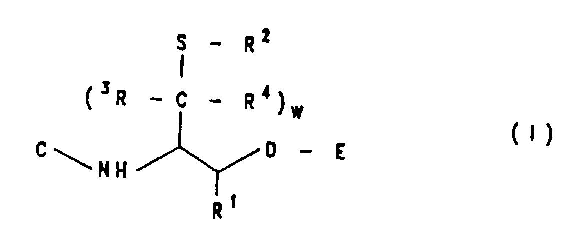 dans laquelle
C est :
a) un atome d'hydrogène
b) un radical choisi dans l'ensemble constitué par :
un résidu aminoacide substitué par le groupe acétyle, la pénicillinamine, un thiophène-aminoacide, Tyr, Asn, Phe, Trp, Nal, D-Tyr, D-Asn ou l'acide gluconique,
D est : un radical choisi dans l'ensemble constitué par : Asp, Asn, D-Asp, D-Asn, Tyr, D-Tyr, la glucosamine ou une liaison covalente,
E est :
a) -NH-(CH₂)ₙ-NR⁵₂,
Les radicaux R⁵ étant identiques ou différents et représentant un radical choisi parmi :
1) un atome d'hydrogène
2) 1 à 6 restes glucidiques ou
3) 1 à 6 restes glucidiques une ou plusieurs fois substitués, indépendamment les uns des autres, par :
3.1 le groupe méthyle,
3.2 un reste glucidique,
3.3 un reste diglucidique,
3.4 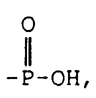
3.5 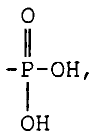
3.6 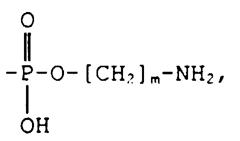
3.7 l'inositol,
3.8 le phosphate d'inositol ou
3.9 un reste phosphate à groupe protecteur de phosphate,
n est un nombre entier allant de 0 à 6,
m est un nombre entier allant de 0 à 4,
b) le reste glycérol
c) -NH- (CH₂)ₘ-R⁶-R⁷,
où R⁶ est :
1) -O-,
2) -CO-O-,
3) 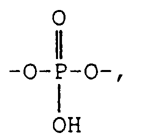
4) 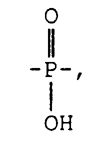
5) -NH-CO-O-,
6) -S-,
7) -SO,
8) -SO₂,
9) -SO₃ ou
10) -O-CO-O-,
où R⁷ représente :
1) un atome d'hydrogène,
2) 1 à 6 restes glucidiques ou
3) 1 à 6 restes glucidiques une ou plusieurs fois substitués, indépendamment les uns des autres, par
3.1 le groupe méthyle,
3.2 un résidu glucidique,
3.3 un résidu diglucidique,
3.4 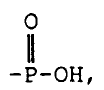
3.5 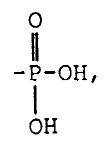
3.6 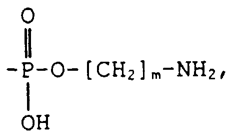
3.7 l'inositol,
3.8 le phosphate d'inositol,
3.9 un reste phosphate à groupe protecteur de phosphate, ou
3.10 -[CH₂]ₘ-CN,
m est un nombre entier allant de 0 à 4,
R¹ est :
a) un groupe alkyle en C₁-C₄,
b) =O ou
c) un groupe -O-alkyle en C₁-C₄,
R² est :
a) un groupe protecteur d'atome de soufre,
b) -SO₂,
c) -SO₃,
d) un groupe alkyle en C₁-C₃,
e) O₂,
f) O₃,
g) 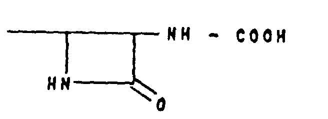 ou
h) un atome d'hydrogène
R³ ou R⁴ représentent, indépendamment l'un de l'autre,
a) un atome d'hydrogène ou
b) le groupe méthyle,
W est le nombre entier 1 ou 2,
formes stéréoisomères, dimères des peptides de formule I avec la cystine en tant que composant de dimérisation, ou sels physiologiquement acceptables du peptide de formule I,
à l'exclusion d'un peptide de formule I dans lequel :
C représente un atome d'hydrogène,
R¹ représente =O,
R² représente un groupe protecteur d'atome de soufre ou un atome d'hydrogène,
R³ et R⁴ représentent chacun un atome d'hydrogène,
E représente -NH-(CH₂)ₘ-R⁶-R⁷
R⁶ représente -CO-O-
R⁷ représente un atome d'hydrogène et
m et w sont chacun 1.

2. Peptide selon la revendication 1, caractérisé en ce que
C est un radical choisi dans l'ensemble constitué par :
un aminoacide substitué par le groupe acétyle, la pénicillinamine, un thiophène-aminoacide, Tyr, Asn, Phe, Trp, Nal, D-Tyr, D-Asn ou l'acide gluconique,
D est un radical choisi dans l'ensemble constitué par :
Asp, Asn, D-Asp, D-Asn, Tyr, D-Tyr, la glucosamine ou une liaison covalente,
E est :
a) -NH-(CH₂)ₙ-NR⁵₂,
les radicaux R⁵ étant identiques ou différents et représentant un radical choisi dans l'ensemble constitué par :
1) un atome d'hydrogène
2) le glucose
3) l'acide gluconique,
4) l'acide galactonique,
5) l'acide mannonique,
6) la glucosamine,
7) le mannose,
8) le fructose,
9) le galactose,
10) le dimannose et
11) le trimannose,
n est un nombre entier allant de 0 à 4, ou
b) -NH-(CH₂)ₘ-R⁶-R⁷,
où R⁶ est
1) -O-,
2) -O-CO-O-,
3) 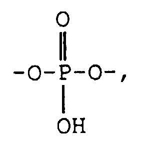
4) -NH-CO-O-,
et où R⁷ est :
1) un atome d'hydrogène ou
2) défini comme en a)1) à a)11),
m est un nombre entier allant de 1 à 3,
R¹ est :
a) =O ou
b) le groupe méthyle,
R² est :
a) un atome d'hydrogène,
b) le groupe méthyle ou
c) -SO₃,
W est le nombre entier 1 ou 2,
formes stéréoisomères ou sels physiologiquement acceptables du peptide de formule I.

3. Peptide selon la revendication 1 ou 2, caractérisé en ce que
C est un résidu choisi parmi les résidus Tyr, Asn, D-Tyr, D-Asn, 2-(2-thiényl)glycine, 3-(2-thiényl)-alanine et Phe,
D est un résidu choisi parmi Asn, Tyr, D-Asn et D-Tyr,
E est un reste d'éthyldiamine ou d'éthanolamine,
W est 1 ou 2,
R¹ est =O,
R² est un atome d'hydrogène ou le groupe méthyle,
formes stéréoisomères ou sels physiologiquement acceptables du peptide de formule I.
